# EUROPEAN PATENT APPLICATION

(11) **EP 0 784 995 A1**
(43) Date of publication of application: **23.07.1997**
(21) Application number: 96850218.7
(22) Date of filing: 20.12.1996
(51) Int. Cl.: A61N 1/05

(54) **Electrode cable**

(30) Priority: 28.12.1995 SE 9504676
(71) Applicant: Pacesetter AB, 171 95 Solna (SE)
(72) Inventor: Lindegren, Ulf, 121 33 Enskededalen (SE); Barsne, Mans, 112 29 Stockholm (SE)
(74) Representative: Winblad, Hans Peter

(57) **Abstract**

The invention relates to an electrode cable (12) intended for implantation in a body cavity. The cable contains at least one elongate electrical conductor (14, 16) whose distal end section (10) has an essentially J-shape in the unrestricted state and a distal end electrode. The conductor (14) forms a channel (18) for the introduction of a stylet for governing cable stiffness and the configuration of the distal end section (10) during implantation. According to the invention, a biodegradable, biocompatible polymer material (26) is arranged on the exterior of at least the J-shaped distal end section (10) of the electrode cable.

## Description

### TECHNICAL FIELD

The present invention relates to an electrode cable intended for implantation in a body cavity, especially for intracardiac stimulation and/or sensing heart signals, containing at least one elongate, flexible electrical conductor with a proximal end and, in the unrestricted state, and essentially J-shaped distal end section with an electrode for fixation to tissue in a heart wall, especially the upper atrial wall, and a channel for insertion of a stylet for governing the stiffness of the electrode cable and the configuration of the distal end section of the electrode cable during implantation.

### THE PRIOR ART

Electrode cables intended for implantation in the right atrium of the heart normally have an essentially J-shaped distal end section to permit the anchoring of the end of the electrode in an upper part of the myocardial wall of the atrium. Here, the J configuration can be pre-shaped to assume a J shape in the unrestricted state, a stylet being introduced into a central channel of the electrode cable in implantation to straighten out the J bend and stiffen the electrode cable during the introductory phase of implantation. The stylet is thereupon withdrawn to permit the distal end section to rebound back into the J shape for fixation of the section in the upper part of the atrium. Examples of such electrode cables are shown and described in US-A-3 890 977 and US-A-4 488 561.

However, these J bends on the distal end of the electrode cable give the distal end section a residual stiffness, causing it to exert increased pressure on the heart wall, pressure which could irritate and damage the heart wall in some instances, thereby raising the threshold value, i.e. the voltage required to make the heart beat.

### SUMMARY OF THE INVENTION

One object of the present invention is to eliminate the said shortcoming and propose an electrode cable which, despite a distal end section with a pre-formed J shape, offers a relatively soft and gentle J shape after implantation.

For this purpose, the aforementioned electrode cable according to the invention is characterized by a biodegradable, biocompatible polymer material, arranged on the exterior of at least the distal, J-shaped end section of the electrode, which before and during implantation is devised to strive to maintain an essentially J-shaped configuration for the distal end section but which dissolves after implantation, through contact with body fluid (blood), in order to create a soft, flexible, distal end section on the electrode cable.

The biodegradable material is appropriately in the form of a tubular sleeve which encloses at least the distal end section of the electrode cable.

Examples of biodegradable, biocompatible polymer materials, which could be used in an application according to the present invention, are stated in the dependent patent claims 4-8.

The invention will now be described in greater detail, referring to the attached drawing.

### FIGURES

FIG. 1 is a schematic view of a pre-shaped, J-shaped distal end section of a bipolar electrode cable; and
FIG. 2 shows a partially opened perspective view of the circled section in FIG. 1.

### PREFERRED EMBODIMENT

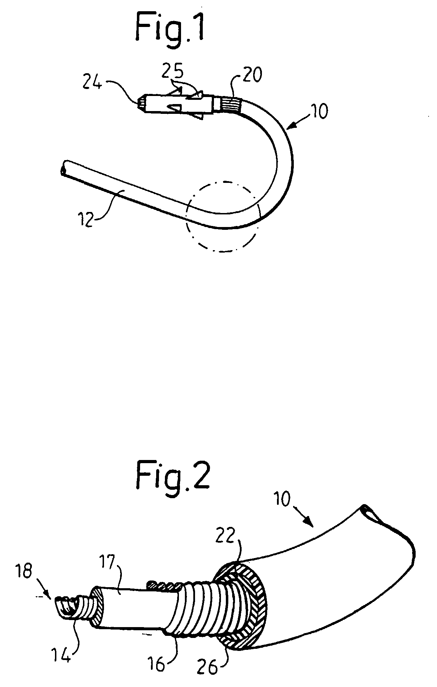

FIG. 1 shows a J-shaped end section 10 of a bipolar electrode cable 12 according to the invention. As shown in detail in FIG. 2, the electrode cable 12 is made of an inner, helically coiled conductor 14 and an outer conductor 16, coaxial to the inner conductor 14, the conductors 14, 16 being separated by an insulating, tubular sleeve 17. The inner conductor 14 forms a central channel 18, intended to admit a stylet (not shown) during implantation in order to stiffen the electrode cable during the latter's introduction through veins into the heart atrium and straighten out the J bend on the distal end section.

The outer conductor 16 is connected to a ring electrode 20 (anode) located at a distance from the tip of the J bend and is enclosed in an insulating sleeve 22 made of e.g. silicone rubber. The inner conductor 14 is connected to an end electrode 24 (cathode) located on the tip ahead of the ring electrode 20 and ahead of anchoring fins 25.

An additional tubular sleeve 26, devised to give the distal end section 10 of the electrode cable 12 an essentially J-shaped configuration in the unrestricted state, is arranged over the sleeve 22. This tubular sleeve 26 is made, according to the present invention, of a biodegradable, biocompatible polymer material, covering at least the distal end section 10, but it could also cover the entire length of the electrode cable 12 from a proximal end to the digital end. This means that the distal J-shaped end section 10 of the outer tubular sleeve 26, after being introduced in the essentially straight state, strives to resume its original J shape, when the stylet is removed from the channel 18 in implantation.

Instead of being devised as a tubular sleeve 26, the biodegradable polymer material can also be devised as bands or strips extending along the exterior of the shell 22, at least in the J-shaped distal end section 10 of the electrode cable, within the scope of the invention.

After a time, when the tip section of the electrode cable 12 has had time to become embedded in the atrial wall, the polymer material in the outer sleeve 26 will have dissolved through contact with blood, thereby leaving behind a soft, flexible electrode cable which is gentle to the heart wall.

The degradation time for the sleeve 26 can range from one day up to one or more months.

The biodegradable polymer material can be selected from e.g. the groups proteins/amino acid polymers, poly(hydroxycarboxyl acids) and/or carbohydrate polymers. The proteins/amino acid polymers group can contain gelatin, collagen, polyserine, polythreonine, polyphenylalanine or the like. The poly(hydroxycarboxyl acids) group can contain polylactides and/or polyglycolides. The carbohydrate polymers group can contain dextran, starch, hyaluronic acid, cellulose or the like.

## Claims

1. An electrode cable intended for implantation in a body cavity, especially for intracardiac stimulation and/or sensing of heart signals, containing at least one elongate, flexible electrical conductor (14, 16) with a proximal end and, in the unrestricted state, an essentially J-shaped distal end section (10) with an electrode (24, 20) for fixation to tissue in a heart wall, especially the upper part of the atrial wall, and a channel (18) for insertion of a stylet for governing cable stiffness and the configuration of the distal end section (10) of the electrode cable during the electrode cable's (12) implantation, **characterized** in that a biodegradable, biocompatible polymer material (26) which, before and during implantation, is arranged to strive to maintain a mainly J-shaped configuration for the distal end section (10) of the electrode cable (10) but which, after implantation, is dissolved by contact with body fluid to make the distal end section (10) soft and flexible, is arranged on the exterior of at least the J-shaped distal end section (10) of the electrode cable (12).

2. An electrode cable according to claim 1, **characterized** in that the biodegradable material is devised as a tubular sleeve (26) which encloses at least the distal end section (10) of the electrode cable (12).

3. An electrode cable according to claim 2, **characterized** in that the tubular sleeve (26) is made of a biodegradable material which covers the entire electrode cable (12) from its proximal end to its distal end.

4. An electrode cable according to any one of claims 1-3, **characterized** in that the polymer material is selected from the groups proteins/amino acid polymers, poly(hydroxycarboxyl acids) and/or carbohydrate polymers.

5. An electrode cable according to claim 4, **characterized** in that the proteins/amino acid polymers group contains gelatin, collagen, polyserine, polythreonine, polyphenylalanine or the like.

6. An electrode cable according to claim 4, **characterized** in that the poly(hydroxycarboxyl acids) group contains polylactides and/or polyglycolides.

7. An electrode cable according to claim 4, **characterized** in that carbohydrate polymers group contains dextran, starch, hyaluronic acid, cellulose or the like.

8. An electrode cable of any of claims 1-7, **characterized** in that the polymer material has a degradation time exceeding at least 24 hours.
